# EUROPÄISCHE PATENTSCHRIFT

(11) **EP 1 888 918 B1**
(45) Veröffentlichungstag und Bekanntmachung des Hinweises auf die Patenterteilung: **07.01.2009**
(21) Anmeldenummer: 06725447.4
(22) Anmeldetag: 30.03.2006
(51) Int. Cl.: F04B 39/10, F04B 39/12, F04B 39/00, F16J 13/06

(54) **KÄLTEMITTELVERDICHTER**
REFRIGERATION COMPRESSOR
COMPRESSEUR DE FLUIDE FRIGORIGENE

(30) Priorität: 31.03.2005 AT 1952005 U
(43) Veröffentlichungstag der Anmeldung: 20.02.2008
(73) Patentinhaber: ACC Austria GmbH, 8280 Fürstenfeld (AT)
(72) Erfinder: BRABEK, Walter, A-8280 Fürstenfeld (AT); ZIPPL, Günther, 7543 Kukmirn (AT)
(74) Vertreter: KLIMENT & HENHAPEL
(86) Internationale Anmeldenummer: PCT/EP2006/061199
(87) Internationale Veröffentlichungsnummer: WO 2006/103279

(56) Entgegenhaltungen:
- WO-A-20/05083268
- DE-A1- 3 514 242
- DE-A1- 3 813 539
- DE-A1- 10 244 565
- US-A- 3 817 564
- US-A- 5 207 564

## Beschreibung

### GEBIET DER ERFINDUNG

Die vorliegende Erfindung bezieht sich auf einen hermetisch gekapselten Kältemittelverdichter mit einem hermetisch dichten Verdichtergehäuse, in dessen Innerem eine ein Kältemittel verdichtende Kolben-Zylinder-Einheit arbeitet, deren Zylindergehäuse mittels einer eine Druckbohrung und eine Saugbohrung aufweisenden Ventilplatte verschlossen ist und ein Saugkanal sowie ein Druckkanal vorgesehenen sind, über welche Kältemittel über ein Saugventil in die Saugbohrung gesaugt sowie über ein Druckventil aus der Druckbohrung in den Druckkanal verdichtet wird, wobei im Saugkanal vorzugsweise ein Saugschalldämpfer angeordnet ist.

Solche Kältemittelverdichter sind seit langem bekannt und kommen vorwiegend in Kühlschränken oder -regalen zum Einsatz. Dementsprechend hoch ist die jährlich produzierte Stückzahl.

### STAND DER TECHNIK

Bei bislang bekannten Kältemittelverdichtern wird die das Zylindergehäuse abschließende Ventilplatte stets mit dem Zylindergehäuse verschraubt, um eine Abdichtung des Zylinderraumes gegenüber dem Inneren des Verdichtergehäuses zu erreichen. Um diese Abdichtung zu erreichen, ist ein definierter Anpressdruck erforderlich. Um die Abdichtung zu erleichtern, ist in vielen Fällen auch eine Schadraumdichtung vorgesehen, welche zwischen Ventilplatte und Zylindergehäuse angeordnet ist und Dichtfunktionen übernimmt.

In der Regel erfolgt die Befestigung der Ventilplatte am Zylindergehäuse über vier Schrauben, die jeweils in den vier Eckbereichen der Ventilplatte angeordnet sind, um einen möglichst gleichmäßigen Anpressdruck zu erreichen.

Die Verwendung von Schrauben bedingt jedoch einen hohen Montageaufwand, da einerseits das Fräsen von Gewindebohrungen in die Ventilplatte und das Zylindergehäuse erforderlich ist und andererseits jede Schraube einzelnen in die vorgesehenen Bohrungen eingeschraubt werden muss. Bei den hohen Stückzahlen, in welchen solche Kältemittelverdichter hergestellt werden, ergibt dies in Summe gesehen einen hohen Zeitanteil an der Fertigungszeit, was wiederum mit hohen Kosten verbunden ist.

Aus der DE 32 21 554 A1 ist etwa ein Kompressorventil mit einer an Führungsständern angeordneten Ventilplatte bekannt, welche mittels eines Federstahlblattes in Position gehalten und am Ende des Zylinderblocks mittels Kopfschrauben befestigt ist.

In der DE 38 13 539 A1 ist eine Auslassventil-Anordnung eines hermetischen Kältemittelkompressors offenbart, wobei ein Blattventil des Ansaugkanals an einem deckelförmigen Rückhalteteil, welches seinerseits durch ein Klemmelement vorgespannt ist, einen Anschlag findet. Dieses Klemmelement ragt mit seinen Endbereichen in Ausnehmungen der Ventilplatte. Diese lediglich zur Vormontage der Ventilkomponenten geeignete Vorrichtungsanordnung macht zur Fixierung des Rückhalteteils noch eine festere Vorspannung erforderlich, indem der Zylinderkopf in axialer Richtung auf das Klemmelement drückt und die Ventilplatte nach wie vor einer eigenen Verschraubung an ihren Endbereichen bedarf, um am Zylindergehäuse befestigt zu werden.

### DARSTELLUNG DER ERFINDUNG

Es ist daher die Aufgabe der vorliegenden Erfindung, diesen Nachteil zu verhindern und einen Kältemittelverdichter der eingangs erwähnten Art vorzuschlagen, welcher in kurzer Zeit montiert werden kann.

Ein weiteres Problem bei bekannten, verschraubten Kältemittelverdichtern ist die unharmonische, punktuelle Krafteinleitung der Dichtkraft in die Ventilplatte.

Ein hermetisch gekapselter kältemitleverdichter mit den Merkmalen des Oberbegriffs von Patentanspruch 1 ist aus der DE 102 44 565 A1 bekannt.

Die vorliegende Erfindung hat auch zum Ziel, einen Kältemittelverdichter zu schaffen, der eine wesentlich harmonischere Krafteinleitung in die Ventilplatte aufweist, bei zumindest gleichbleibender Dichtwirkung zwischen Ventilplatte und Zylindergehäuse.

Erfindungsgemäß wird dies durch die kennzeichnenden Merkmale des Anspruchs 1 erreicht.

Durch das Vorsehen eines kreisringförmigen Klemmelementes, welches die Ventilplatte zumindest entlang eines Abschnitts deren Umfangs an das Zylindergehäuse klemmt, ist einerseits die aufwändige Montage mittels Schrauben nicht mehr erforderlich und andererseits erfolgt eine harmonische Krafteinleitung in die Ventilplatte, wodurch eine sichere Abdichtung des Zylinderinnenraums gegenüber dem Inneren des Verdichtergehäuses gegeben ist.

Gemäß einer bevorzugten Ausführungsvariante ist das Klemmelement im wesentlichen J-förmig ausgebildet und ist mit einem Endabschnitt an einer am Zylindergehäuse vorgesehen Hinterschneidung verrastet, wobei der andere Endabschnitt des Klemmelementes einen ersten Klemmschenkel bildet der das Zylindergehäuse gegen die Ventilplatte presst.

3 Gemäß den kennzeichnenden Merkmalen der Ansprüche 3 und 4 kann zwischen dem die Ventilplatte klemmenden Klemmschenkel und der Ventilplatte ein das Druckventil in Form einer Druckblattfeder formendes Trägerelement und/oder ein Dichtelement, welche beide flächig auf der Ventilplatte aufliegen, angeordnet sein.

Gemäß den kennzeichnenden Merkmalen des Anspruchs 5, weist das Zylindergehäuse einen Absatz auf, in welchem die Ventilplatte zumindest teilweise versenkt ist, wobei gemäß einer in den kennzeichnenden Merkmalen des Anspruchs 6 beschriebenen bevorzugten Ausführungsform jene dem Kolben abgewandte Oberfläche der Ventilplatte bündig mit dem Zylindergehäuse abschließt.

Gemäß einer weiteren alternativen Ausführungsform wie in Anspruch 7 beschrieben, ist der Zylinderkopf durch den Druckkanal bzw. den Saugkanal bildenden Bauteile gebildet, und das Klemmelement weist einen weiteren Klemmschenkel auf, welcher Druckkanal und Saugkanal an die Ventilplatte bzw. in die Saug - bzw. Druckbohrung presst.

Gemäß den kennzeichnenden Merkmalen des Anspruchs 8 ist bei einer anderen alternativen Ausführungsvariante ein weiteres Klemmelement vorgesehen, welches am Klemmelement verrastbar ist und den Zylinderkopf bestehend aus den Druckkanal bzw. den Saugkanal bildenden Bauteilen an die Ventilplatte bzw. in die Saugbohrung und/oder die Druckbohrung klemmt.

Die kennzeichnenden Merkmale der Ansprüche 9 und 10 beschreiben alternative Ausführungsvarianten des Klemmelementes in Zusammenhang mit einem herkömmlichen Zylinderkopf in Form eines Zylinderdeckels.

### KURZE BESCHREIBUNG DER FIGUREN

Im Anschluss erfolgt nun eine detaillierte Beschreibung der Erfindung anhand von Ausführungsbeispielen.

Dabei zeigt:
- Fig.1: eine Schnittansicht eines Kältemittelverdichters nach dem Stand der Technik
- Fig.2: eine Schnittansicht einer Zylindergehäuse-Ventilplatten-Einheit mit erfindungsgemäßem Klemmelement in Richtung der Pfeile DD aus Fig.4
- Fig.2a: eine Detailansicht einer Zylindergehäuse-Ventilplatteneinheit mit erfindungsgemäßem Klemmelement
- Fig.3: eine axonometrische Ansicht der Zylindergehäuse-Ventilplatten-Einheit gemäß Fig.2
- Fig.4: eine Draufsicht auf eine Zylindergehäuse-Ventilplatten-Einheit gemäß den Fig.3 und 4
- Fig.5: eine axonometrische Ansicht einer alternativen Ausführung der Zylindergehäuse-Ventilplatten-Einheit aus den Fig. 2 bis 4
- Fig.6: eine Ansicht einer weiteren alternativen Zylindergehäuse-Ventilplatten-Einheit mit erfindungsgemäßem Klemmelement
- Fig.7: eine teilweise geschnittene Ansicht in Richtung der Pfeile A aus Fig.6
- Fig.8: eine Schnittansicht entlang der Ebene B aus Fig.6
- Fig.9: eine Schnittansicht einer zusätzlichen alternativen Zylindergehäuse-Ventilplatten-Einheit mit erfindungsgemäßem Klemmelement
- Fig.10: eine axonometrische Ansicht einer anderen alternativen Zylindergehäuse-Ventilplatten-Einheit mit erfindungsgemäßem Klemmelement

### WEGE ZUR AUSFÜHRUNG DER ERFINDUNG

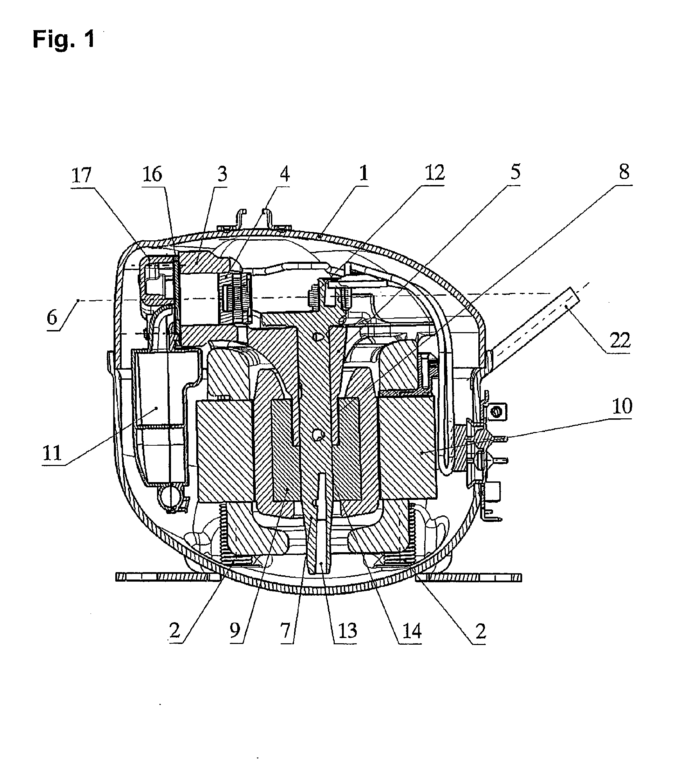

Fig.1 zeigt eine Schnittansicht durch einen hermetisch gekapselten Kältemittelverdichter wie er aus dem Stand der Technik bekannt ist. Im Inneren eines hermetisch abdichtenden Verdichtergehäuses 1 ist eine Zylindergehäuse-Ventilplatten-Einheit samt Motor und Kolben über Federn 2 elastisch gelagert.

Die Zylindergehäuse-Ventilplatten-Einheit samt Motor und Kolben besteht im wesentlichen aus einem Zylindergehäuse 3 sowie dem darin eine Hubbewegung vollführenden Kolben 4, sowie einem Kurbelwellenlager 5, welches senkrecht zur Zylinderachse 6 angeordnet ist. Das Kurbelwellenlager 5 nimmt eine Kurbelwelle 7 auf und ragt in eine zentrische Bohrung 8 des Rotors 9 eines Elektromotors 10. Am oberen Ende der Kurbelwelle 7 befindet sich ein Pleuellager 12, über das ein Pleuel und in weiterer Folge der Kolben 4 angetrieben wird. Die Kurbelwelle 7 weist eine Schmierölbohrung 13 auf und ist im Bereich 14 am Rotor 9 fixiert. Am Zylinderkopf ist der Saugschalldämpfer 11 angeordnet, der beim Ansaugvorgang des Kältemittels die Geräuschentwicklung auf ein Minimum reduzieren soll.

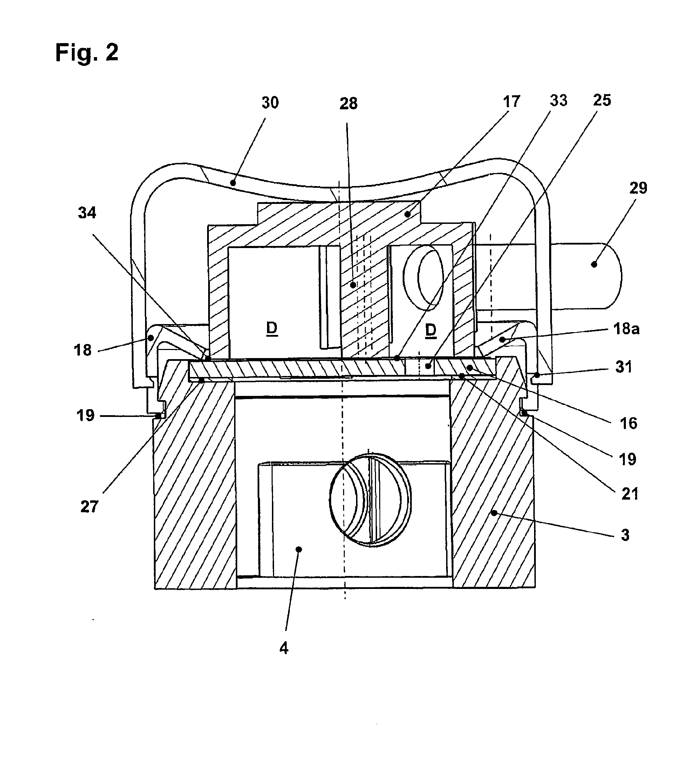

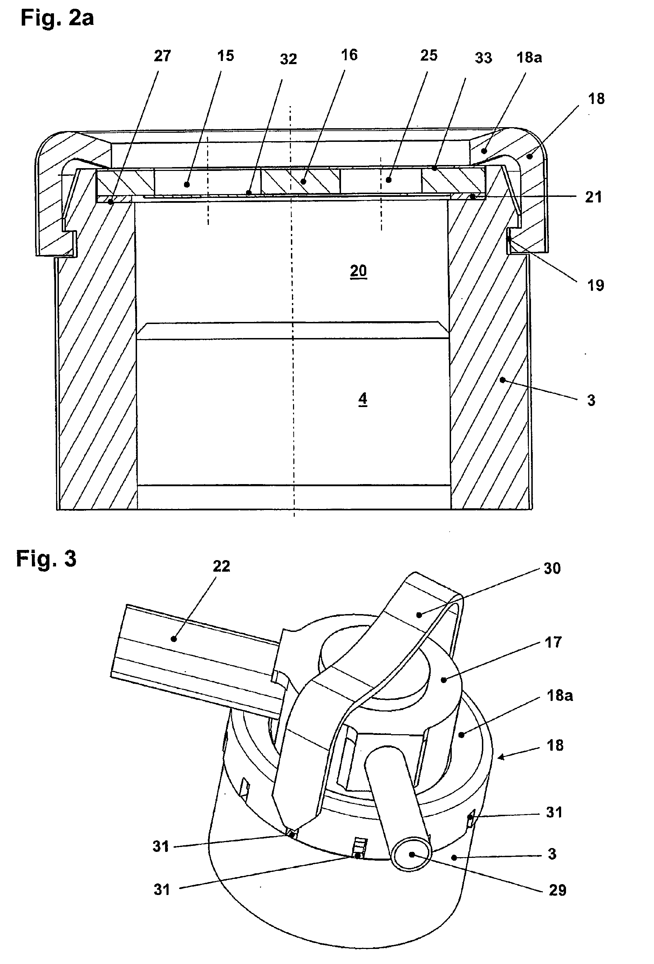

Das Zylindergehäuse 3 ist an seiner Stirnseite mittels einer Ventilplatte 16 abgeschlossen, die bei herkömmlichen Kältemittelverdichtern mit dem Zylindergehäuse verschraubt ist. Im Anschluss an die Ventilplatte 16 ist bei Kältemittelverdichtern nach dem Stand der Technik eine Zylinderdeckel 17 angeordnet, der an seiner der Ventilplatte 16 zugewandten Seite mit Stegen 28 (siehe Fig.2) versehen ist, welche in Kombination mit den Seitenwänden des Zylinderdeckels 17 und der Ventilplatte 16 einen Druckkanal bilden, über welchen vom Kolben aus dem Zylindergehäuse 3 ausgeschobenes, verdichtetes Kältemittel in Druckleitungen 29 weitergeleitet und aus dem hermetisch dichten Verdichtergehäuse 1 befördert wird. Der Zylinderdeckel 17 ist in der Regel ebenfalls mit dem Zylindergehäuse 3 verschraubt, so dass Zylinderdeckel 17, Ventilplatte 16 und Zylindergehäuse 3 gemeinsam verschraubt sind.

Erfindungsgemäß ist jedoch vorgesehen, dass die Verbindung zwischen Ventilplatte 16 und Zylindergehäuse 3 nicht mittels Verschraubung erfolgt sondern aufgrund einer Klemmung.

Fig.2 zeigt eine Schnittansicht einer an sich bekannten Zylindergehäuse 3 -Ventilplatten 16 - Einheit, jedoch mit erfindungsgemäßer Klemmung. Die Ventilplatte 16 und der Zylinderdeckel 17, begrenzen gemeinsam einen Druckkanal, über welchen verdichtetes Kältemittel, welches über eine Druckbohrung 25 und ein Druckventil 33 in den Druckkanal und weiter in eine Druckleitung 29 befördert wird. Die Saugbohrung ist in dieser Ansicht nicht sichtbar.

Im Gegensatz zu der in Fig.1 gezeigten Zylindergehäuse 3 - Ventilplatten 16 - Einheit, bei welcher der Zylinderdeckel 17 mit der Ventilplatte 16 und diese mit dem Zylindergehäuse 3 verschraubt ist (Schrauben in Fig.1 nicht sichtbar), ist bei einer Zylindergehäuse 3 - Ventilplatten 16 - Einheit wie in Fig.2 offenbart, die Ventilplatte 16 mittels eines Klemmelementes 18 am Zylindergehäuse 3 festgeklemmt.

Zu diesem Zweck weist das Zylindergehäuse 3 eine Hinterschneidung 19 auf, welche mit einem Endabschnitt des Klemmelementes 18 verrastbar ist. Das Klemmelement 18 selbst weist weiters einen ersten Klemmschenkel 18a auf, der die Ventilplatte 16 entlang deren gesamten Umfang gegen das Zylindergehäuse 3 klemmt, welches zu diesem Zweck einen Absatz 27 aufweist, in welchem die Ventilplatte 16 zumindest teilweise, vorzugsweise jedoch gänzlich versenkt ist, wodurch gleichzeitig eine Zentrierung der Ventilplatte erreicht wird. Die Klemmung erfolgt dabei entlang einer Kontaktkante des Klemmschenkels 18a, die in diesem Ausführungsbeispiel, ebenso wie die Ventilplatte 16 eine kreisrunde, geschlossene Form aufweist. Für den Fachmann ist jedoch offensichtlich, dass die Form der Kontaktkante, welche schlussendlich die Klemmung der Ventilplatte 16 bewirkt, eine beliebige sein kann. Aber auch das Klemmelement 18 selbst kann beliebige Form haben, solange nur gewährleistet ist, dass eine Klemmung der Ventilplatte 16 gegen das Zylindergehäuse 3 mit ausreichender Klemmkraft erfolgt. Eine ausreichende Klemmkraft ist dann gewährleistet, wenn sie den Zylinderinnenraum 20 gegen das das Zylindergehäuse 3 umgebende, Innere des Verdichtergehäuses 1 abdichtet. Dies wird in der Praxis durch eine Klemmung entlang des Umfangs der Ventilplatte 16 erreicht, wobei eine Klemmung entlang des gesamten Umfanges erfindungsgemäß nicht zwingend erforderlich ist und es, je nach Klemmkraft, durchaus auch ausreichend sein kann, lediglich einen Abschnitt des Umfangs der Ventilplatte 16 mittels der Klemmschenkel 18a zu klemmen. Wichtig ist jedoch, dass die Klemmung entlang des Umfangs, also im Umfangsbereich der Ventilplatte 16 erfolgt. Um die Dichtwirkung zu verbessern, ist in vielen Fällen eine Schadraumdichtung 21 vorgesehen.

Da das Klemmelement 18 für die Klemmung der Ventilplatte 16 gegen das Zylindergehäuse vorgesehen ist, ist erfindungsgemäß ein weiteres Klemmelement . 30 vorgesehen, dass den Zylinderdeckel 17 an die Ventilplatte 16 klemmt. Das weitere Klemmelement 30 ist zu diesem Zweck in zwei am Klemmelement 18 vorgesehene, einander diametral gegenüberliegenden Nuten oder Öffnungen 31 eingehängt und überspannt den Zylinderdeckel 17 bei gleichzeitiger Ausübung einer Klemmkraft auf den Zylinderdeckel 17 im Bereich der Achse der Kolbenbohrung.

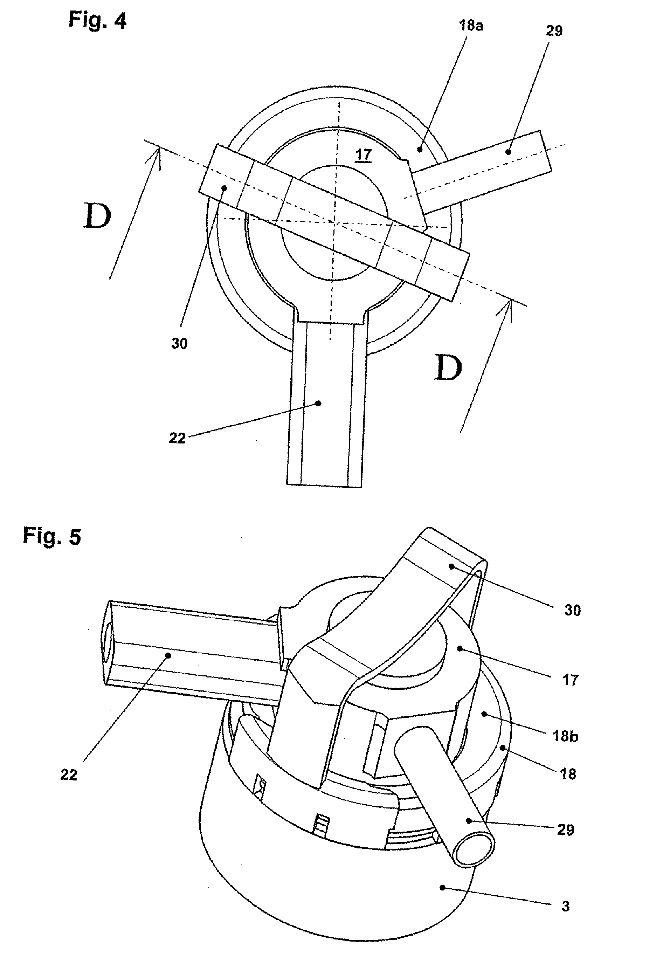

Fig.3 und 4 zeigen zwei weitere Ansichten der Zylindergehäuse 3 -Ventilplatten 16 - Einheit aus Fig.2. In Fig.4 ist zusätzlich zur Druckleitung 29 auch die Saugleitung 22 sichtbar.

Fig.5 zeigt eine axonometrische Ansicht einer alternativen Ausführung der Zylindergehäuse 3 -Ventilplatten 16 - Einheit aus den Fig. 2 bis 4. Dabei ist das Klemmelement 18 und das Klemmelement 30 einstückig ausgeführt. In einer bevorzugten Ausführungsvariante sind die beiden Klemmelemente 18 und 30 miteinander verschweißt. Des weiteren klemmt das Klemmelement 18 die Ventilplatte lediglich entlang eines Abschnittes des Umfangs, also nicht über den gesamten Umfang, wobei die Klemmkraft so gewählt ist, dass eine ausreichende Abdichtung des Zylinderinnenraums 20 gegen das das Zylindergehäuse 3 umgebende, Innere des Verdichtergehäuses 1 erfolgt.

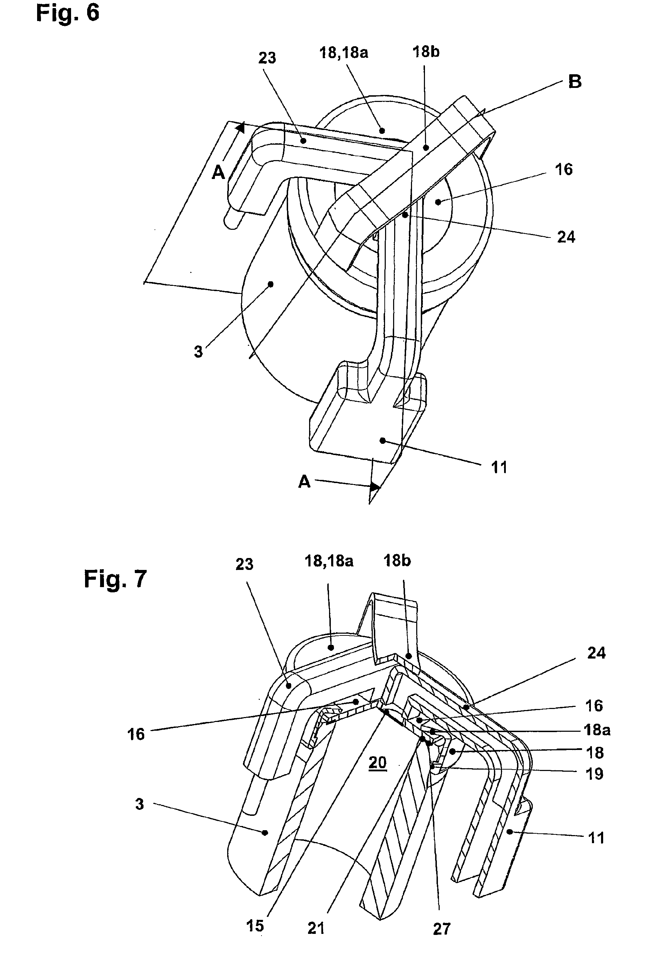

Fig.6,7 und 8 zeigen eine weitere alternative Ausführungsvariante einer Zylindergehäuse 3 - Ventilplatten 16 - Einheit. Das Klemmelement 18 ist wieder mit einer am Zylindergehäuse 3 angeordneten Hinterschneidung 19 verrastet. Ein erster Klemmschenkel 18a klemmt die Ventilplatte 16 entlang deren gesamten Umfang gegen das Zylindergehäuse 3, welches zu diesem Zweck eine Absatz 27 aufweist, in welchem die Ventilplatte 16 zumindest teilweise versenkt ist. Die Klemmung erfolgt dabei wieder vorzugsweise entlang einer Kontaktkante des Klemmschenkels 18a.

Im Unterschied zu der in Fig.2 gezeigten Zylindergehäuse 3 - Ventilplatten 16 - Einheit, welche einen herkömmlichen Zylinderkopf in Form eines Zylinderdeckels 17 sowie einem an der Ventilplatte 16 befestigten Saugkanal (in Fig.2 nicht sichtbar) zum Saugschalldämpfer 11 aufweist, besteht der in den Fig.6,7 und 8 gezeigte Zylinderkopf aus zwei Bauteilen 23,24, welche jeder eigenständig so ausgebildet ist, dass er einen unabhängigen Strömungskanal für das Kältemittel bildet, wobei ein Strömungskanal 23 den Druckkanal bildet und ein Strömungskanal 24 den Saugkanal, wobei die Ventilplatte nicht als Abschnittsweise Begrenzung des Druckkanals eingesetzt wird, wie das bei bekannten Kältemittelverdichtern gemäß Fig.2 der Fall ist. Dementsprechend ist der Druckkanal 23 mit der in der Ventilplatte 16 vorgesehenen Druckbohrung 25 dicht verbunden und der Saugkanal 24 mit der ebenfalls in der Ventilplatte 16 vorgesehenen Saugbohrung 15 dicht verbunden. Der den Saugkanal bildende Bauteil 24 ist weiters mit dem an sich bekannten Saugschalldämpfer 11 verbunden.

Während der Saugkanal bereits bei bekannten Kältemittelverdichtern als eigenständiger Strömungskanal ausgebildet war, hat die Ausbildung eines eigenständigen Strömungskanals für das verdichtete, heiße Kältemittel in Form eines eigenen Bauteils 23 zur Folge, dass der Einsatz eines herkömmlichen Zylinderdeckels wie in der Fig.1 und Fig.2 mit 17 bezeichnet, nicht mehr erforderlich ist. Da beim Einsatz herkömmlicher Zylinderdeckel 17 die Ventilplatte 16 an Ihrer dem Kolben abgewandten Oberfläche stets entweder in direktem Kontakt mit dem verdichteten Kältemittel war (da sie Bestandteil des Druckkanals war) bzw. zumindest in direktem Kontakt mit dem den Druckkanal bildenden Bauteil war, konnte bei herkömmlichen Kältemittelverdichtern das verdichtete, heiße Kältemittel seine Wärme nicht nur aus dem Inneren des Zylindergehäuses an die Ventilplatte 16 abgeben, sondern auch aus dem Äußeren, eben an jene dem Kolben abgewandte Oberfläche der Ventilplatte 16.

Durch das Vorsehen zweier eigenständiger Bauteile 23,24, die in ihrem unmittelbar an die Ventilplatte 16 anschließenden Abschnitt, im wesentlichen rechtwinkelig von dieser abstehend weggeführt werden, kann das verdichtete, heiße Kältemittel sofort von der Ventilplatte 16 wegtransportiert werden, ohne die Ventilplatte 16 an Ihrer dem Kolben abgewandten Oberfläche zu kontaktieren, bzw. diese Oberfläche lediglich an einem kleinen Abschnitt rund um die Druckbohrung 25, so dass ein Wärmeübergang vom verdichteten, heißen Kältemittel auf die Ventilplatte über diese Oberfläche vermieden werden kann, wobei vorgesehen sein kann, dass im Bauteil 23 auch noch das Druckventil 33 in Form einer Druckblattfeder angeordnet ist.

Hinzu kommt, dass die beiden Bauteile 23,24 komplett aus Kunststoff gefertigt werden können, so dass auch der Wärmeübergang von diesen Bauteilen auf die Ventilplatte praktisch verhindert werden kann.

Um eine ausreichend dichte Verbindung zwischen dem Druckkanal und dem Saugkanal bzw. den Bauteilen 23,24 mit der Saugbohrung 15 bzw. Druckbohrung 25 in der Ventilplatte 16 zu ermöglichen, ist erfindungsgemäß vorgesehen, dass das Klemmelement 18 zusätzlich zu seinem ersten Klemmschenkel 18a, der die Ventilplatte 16 in den Absatz 27 des Zylindergehäuses 3 klemmt, einen weiteren Klemmschenkel 18b aufweist, der die beiden Bauteile 23,24 gegen die Ventilplatte 16 bzw. in die Druckbohrung 25 bzw. Saugbohrung 15 presst.

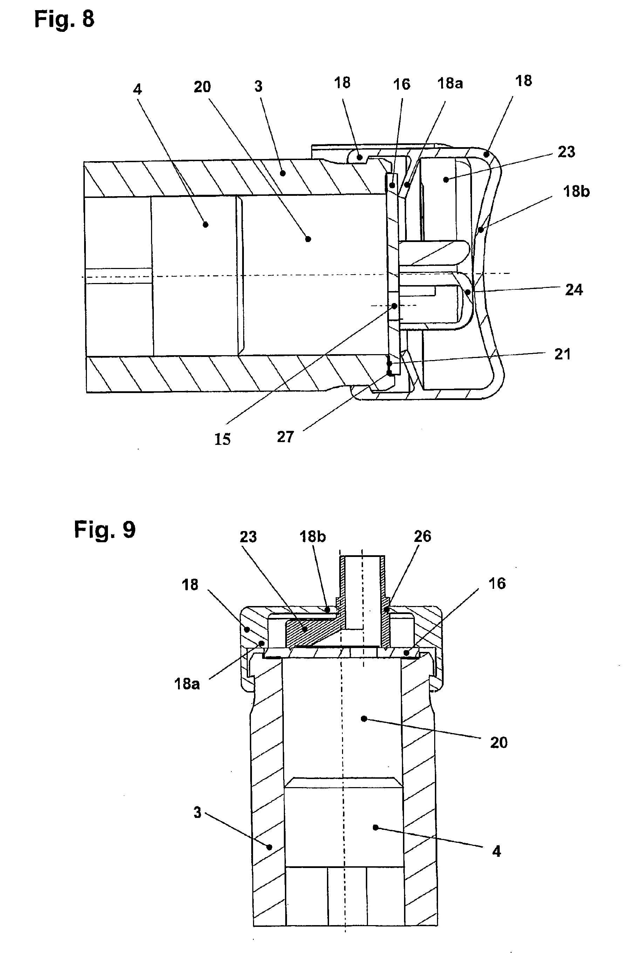

Fig.9 zeigt eine zusätzliche alternative Zylindergehäuse 3 - Ventilplatten 16 - Einheit, wobei das erfindungsgemäße Klemmelement 18 wiederum zwei Klemmabschnitte 18a,18b aufweist. Der Klemmabschnitt 18b verrastet dabei mit einer Nut 26 am den Druckkanal bildenden Bauteil 23, sowie am den Saugkanal bildenden Bauteil 24 (nicht sichtbar).

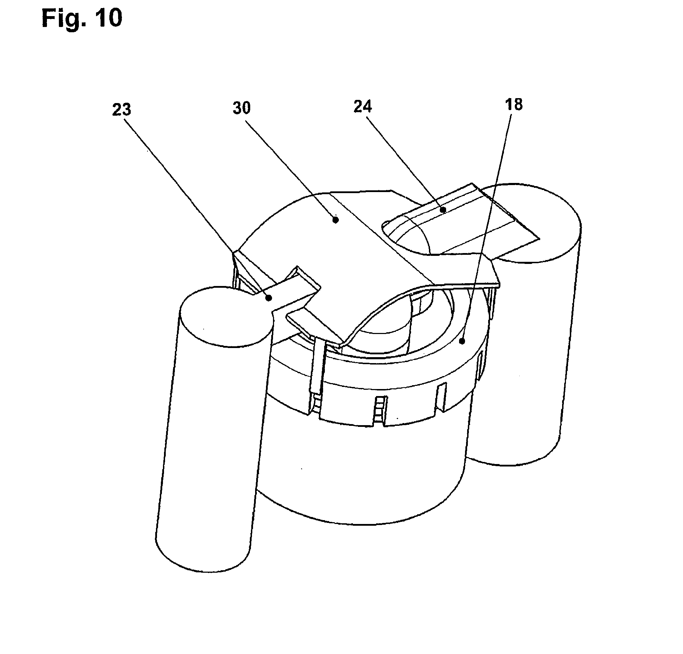

Aufgrund der Ausführungsbeispiele ist es für den Fachmann deutlich, dass die Art und Weise der Ausbildung des Fig.10 zeigt eine axonometrische Ansicht einer anderen alternativen Zylindergehäuse 3 -Ventilplatten 16 - Einheit mit erfindungsgemäßem Klemmelement 18, jedoch mit neuer Kopfgruppe, also mit einem den Druckkanal bildenden Bauteil 23 und einem den Saugkanal bildenden Bauteil 24. Im Gegensatz zu der in den Fig.6,7,8 8 offenbarten Zylindergehäuse 3 - Ventilplatten 16 - Einheit, erfolgt die Klemmung der Bauteile 23,24 hier jedoch mittels eines separaten Klemmelementes 30, das in am Klemmelement 18 angeordneten Nuten bzw. Öffnungen 31 verrastet ist.

Zylinderkopfes, also ob mit Zylinderdeckel 17, wie bisher bekannt, oder aber mit einer Kopfgruppe wie in den Fig.6 bis 10 beschrieben, auf die Erfindung keinen Einfluss hat, da es das Ziel der Erfindung ist, die Ventilplatte 16 gegen das Zylindergehäuse 3 zu klemmen um das montageaufwändige Verschrauben zu vermeiden.

### Bezugszeichenliste

1. Verdichtergehäuse
2. Federn
3. Zylindergehäuse
4. Kolben
5. Kurbelwellenlager
6. Zylinderachse
7. Kurbelwelle
8. zentrische Bohrung
9. Rotor
10. Elektromotor
11. Saugschalldämpfer
12. Pleuellager
13. Schmierölbohrung
14. Fixierungsbereich
15. Saugbohrung
16. Ventilplatte
17. Zylinderdeckel
18. Klemmelement
19. Hinterschneidung
20. Zylinderinnenraum
21. Schadraumdichtung
22. Saugleitung
23. den Druckkanal bildender Bauteil
24. den Saugkanal bildender Bauteil
25. Druckbohrung
26. Nut
27. Absatz
28. Steg
29. Druckleitung
30. Klemmelement
31. Nut oder Öffnung
32. Saugblattfeder
33. Druckblattfeder
34. Dichtung Druckseite

## Patentansprüche

1. Hermetisch gekapselter Kältemittelverdichter mit einem hermetisch dichten Verdichtergehäuse, in dessen Innerem eine ein Kältemittel verdichtende Kolben-Zylinder-Einheit arbeitet, deren Zylindergehäuse (3) mittels einer eine Druckbohrung (25) und eine Saugbohrung (15) aufweisenden Ventilplatte (16) verschlossen ist und ein Saugkanal sowie ein Druckkanal vorgesehenen sind, über welche Kältemittel über ein Saugventil in die Saugbohrung (15) gesaugt sowie über ein Druckventil aus der Druckbohrung (25) in den Druckkanal verdichtet wird, wobei im Saugkanal vorzugsweise ein Saugschalldämpfer (11) angeordnet ist, **dadurch gekennzeichnet, dass** ein kreisringförmiges Klemmelement (18) vorgesehen ist, welches die Ventilplatte (16) mittels eines Klemmschenkels (18a) zumindest entlang eines Abschnittes deren Umfangs, vorzugsweise jedoch entlang des gesamten Umfangs kontaktiert und an das Zylindergehäuse (3) klemmt.

2. Hermetisch gekapselter Kältemittelverdichter nach Anspruch 1, **dadurch gekennzeichnet, dass** das Klemmelement (18) einen im wesentlichen J-förmigen Querschnitt aufweist.

3. Hermetisch gekapselter Kältemittelverdichter nach einem der Ansprüche 1 oder 2, **dadurch gekennzeichnet, dass** am Zylindergehäuse (3) Hinterschneidungen (19) vorgesehen sind, welche mit einem Endabschnitt des Klemmelementes (18) verrastbar sind.

4. Hermetisch gekapselter Kältemittelverdichter nach Anspruch 1 oder 2 , **dadurch gekennzeichnet, dass** zwischen Ventilplatte (16) und Klemmschenkel (18a) ein das Druckventil (33), vorzugsweise in Form einer Druckblattfeder, formendes Trägerelement, welches flächig auf der Ventilplatte (16) aufliegt, angeordnet ist.

5. Hermetisch gekapselter Kältemittelverdichter nach einem der Ansprüche 1 bis 3, **dadurch gekennzeichnet, dass** zwischen Ventilplatte (16) und Klemmschenkel (18a) ein flächig auf der Ventilplatte (16) aufliegendes Dichtelement (34) angeordnet ist.

6. Hermetisch gekapselter Kältemittelverdichter nach einem der Ansprüche 1 bis 5, **dadurch gekennzeichnet, dass** das Zylindergehäuse einen Absatz (27) aufweist, in welchem die Ventilplatte (16) zumindest teilweise versenkt ist.

7. Hermetisch gekapselter Kältemittelverdichter nach einem der Ansprüche 1 bis 6, **dadurch gekennzeichnet, dass** jene dem Kolben (4) abgewandte Oberfläche der Ventilplatte (16) bündig mit dem Zylindergehäuse (13) abschließt.

8. Hermetisch gekapselter Kältemittelverdichter nach einem der Ansprüche 1 bis 6, **dadurch gekennzeichnet, dass** das Klemmelement (18) zumindest einen weiteren Klemmschenkel (18b) aufweist, welcher einen den Druckkanal bzw. den Saugkanal bildenden Bauteil (23,24) an die Ventilplatte (16) bzw. in die Saugbohrung (15) und/oder die Druckbohrung (25) klemmt.

9. Hermetisch gekapselter Kältemittelverdichter nach einem der Ansprüche 1 bis 7, **dadurch gekennzeichnet, dass** ein weiteres Klemmelement (30) vorgesehen ist, welches am Klemmelement (18) verrastbar ist und einen den Druckkanal bzw. den Saugkanal bildenden Bauteil (23,24) an die Ventilplatte (16) bzw. in die Saugbohrung (15) und/oder die Druckbohrung (25) klemmt.

10. Hermetisch gekapselter Kältemittelverdichter nach einem der Ansprüche 1 bis 9, **dadurch gekennzeichnet, dass** das Klemmelement (18) zumindest einen weiteren Klemmschenkel (18b) aufweist, welcher einen Zylinderdeckel (17) gegen die Ventilplatte (16) klemmt.

11. Hermetisch gekapselter Kältemittelverdichter nach einem der Ansprüche 1 bis 9, **dadurch gekennzeichnet, dass** ein weiteres Klemmelement (30) vorgesehen ist, welches am Klemmelement (18) verrastbar ist und einen Zylinderdeckel (17) gegen die Ventilplatte (16) klemmt.

## Claims

1. A hermetically encapsulated refrigerant compressor, comprising a hermetically sealed compressor housing, in the interior of which a piston-cylinder unit works which compresses a refrigerant, whose cylinder housing (3) is sealed by means of a valve plate (16) comprising a pressure bore (25) and an intake bore (15), and an intake duct and a pressure duct are provided through which refrigerant is drawn into the intake bore (15) via a suction valve and is compressed via a pressure valve from the pressure bore (25) into the pressure duct, preferably with a muffler (11) being arranged in the intake duct, **characterized in that** a clamping element (18) in the form of an annulus is provided which clamps the valve plate (16) via a clamping leg (18a) at least along a section of its circumference, preferably along the entire circumference, against the cylinder housing (3).

2. A hermetically encapsulated refrigerant compressor according to claim 1, **characterized in that** the clamping element (18) comprises a substantially J-shaped cross section.

3. A hermetically encapsulated refrigerant compressor according to one of the claims 1 to 3, **characterized in that** undercuts (19) are provided on the cylinder housing (3) which can be latched together with an end section of the clamping element (18).

4. A hermetically encapsulated refrigerant compressor according to claim 5, **characterized in that** a carrier element forming the pressure valve (33) - preferably in the form of a pressure leaf spring - which rests in a planar manner on the valve plate (16) is arranged between the clamping legs (18a) and the valve plate (16).

5. A hermetically encapsulated refrigerant compressor according to claim 5 or 6, **characterized in that** a sealing element (34) which rests in a planar manner on the valve plate (16) is arranged between the valve plate (16) and the clamping legs (18a).

6. A hermetically encapsulated refrigerant compressor according to one of the claims 1 to 7, **characterized in that** the cylinder housing comprises a shoulder (27) in which the valve plate (16) is sunk at least partly.

7. A hermetically encapsulated refrigerant compressor according to one of the claims 1 to 8, **characterized in that** the surface of the valve plate (16) averted from the piston (4) is flush with the cylinder housing (13).

8. A hermetically encapsulated refrigerant compressor according to one of the claims 5 to 9, **characterized in that** the clamping element (18) comprises at least one further clamping leg (18b) which clamps a component (23, 24) forming the pressure duct or intake duct against the valve plate (16) or in the intake bore (15) and/or the pressure bore (25).

9. A hermetically encapsulated refrigerant compressor according to one of the claims 1 to 9, **characterized in that** a further clamping element (30) is provided which can be latched to the clamping element (18) and clamps the component (23, 24) forming the pressure duct or the intake duct against the valve plate (16) or in the intake bore (15) and/or the pressure bore (25).

10. A hermetically encapsulated refrigerant compressor according to one of the claims 5 to 11, **characterized in that** the clamping element (18) comprises at least one further clamping leg (18b) which clamps a cylinder cover (17) against the valve plate (16).

11. A hermetically encapsulated refrigerant compressor according to one of the claims 5 to 12, **characterized in that** a further clamping element (30) is provided which can be latched to the clamping element (18) and clamps a cylinder cover (17) against the valve plate (16).

## Revendications

1. Compresseur de réfrigérant encapsulé hermétiquement avec un boîtier de compresseur hermétique à l'intérieur duquel fonctionne une unité de piston et cylindre compressant un fluide réfrigérant, donc le corps de cylindre (3) est fermé au moyen d'une plaque de vanne (16) présentant un alésage de pression (25) et un alésage d'aspiration (15) et il est prévu un canal d'aspiration ainsi qu'un canal de pression, par lesquels le réfrigérant est aspiré via une soupape d'aspiration dans l'alésage d'aspiration (15) et compressé via une soupape de pression hors de l'alésage de pression (25) vers le canal de pression, le canal d'aspiration contenant de préférence un élément insonorisant d'aspiration (11), **caractérisé en ce qu'**il est prévu un élément de serrage (18) en forme d'anneau circulaire, qui vient en contact avec la plaque de vanne (16) au moyen d'un bras de serrage (18a) au moins sur une partie de la circonférence de celle-ci, mais de préférence sur toute sa circonférence, et la serre sur le corps de cylindre (3).

2. Compresseur de réfrigérant encapsulé hermétiquement selon la revendication 1, **caractérisé en ce que** l'élément de serrage (18) a une section sensiblement en forme de J.

3. Compresseur de réfrigérant encapsulé hermétiquement selon l'une des revendications 1 ou 2, **caractérisé en ce qu'**il est prévu sur le corps de cylindre (3) des contre-dépouilles (19) qui peuvent être engagées avec une partie d'extrémité de l'élément de serrage (18).

4. Compresseur pour réfrigérant encapsulé hermétiquement selon la revendication 1 ou 2, **caractérisé en ce qu'**il est prévu disposé entre la plaque de vanne (16) et le bras de serrage (18a) un élément portant qui forme la soupape de pression (33), de préférence sous la forme d'un ressort à lames de compression, qui repose à plat sur la plaque de vanne (16).

5. Compresseur de réfrigérant encapsulé hermétiquement selon l'une des revendications 1 à 3, **caractérisé en ce qu'**il est prévu disposé entre la plaque de vanne (16) et le bras de serrage (18a) un élément d'étanchéité (34) reposant à plat sur la plaque de vanne (16).

6. Compresseur pour réfrigérant encapsulé hermétiquement selon l'une des revendications 1 à 5, **caractérisé en ce que** le corps de cylindre présente un décrochement (27) dans lequel la plaque de vanne (16) est au moins partiellement enfoncée.

7. Compresseur pour réfrigérant encapsulé hermétiquement selon l'une des revendications 1 à 6, **caractérisé en ce que** la surface de la plaque de vanne (16) tournée à l'opposé du piston (4) affleure de niveau avec le corps de cylindre (13).

8. Compresseur de réfrigérant encapsulé hermétiquement selon l'une des revendications 1 à 6, **caractérisé en ce que** l'élément de serrage (18) présente au moins un autre bras de serrage (18b) qui serre une pièce (23, 24) formant le canal de pression ou le canal d'aspiration sur la plaque de vanne (16) ou dans l'alésage d'aspiration (15) et/ou l'alésage de pression (25).

9. Compresseur de réfrigérant encapsulé hermétiquement selon l'une des revendications 1 à 7, **caractérisé en ce qu'**il est prévu un autre élément de serrage (30) qui peut être emboîté sur l'élément de serrage (18) et serre une pièce (23, 24) formant le canal de pression ou le canal d'aspiration sur la plaque de vanne (16) ou dans l'alésage d'aspiration (15) et/ou l'alésage de pression (25).

10. Compresseur de réfrigérant encapsulé hermétiquement selon l'une des revendications 1 à 9, **caractérisé en ce que** l'élément de serrage (18) présente au moins un autre bras de serrage (18b) qui serre un couvercle de cylindre (17) contre la plaque de vanne (16).

11. Compresseur de réfrigérant encapsulé hermétiquement selon l'une des revendications 1 à 9, **caractérisé en ce qu'**il est prévu un autre élément de serrage (30) qui peut être emboîté sur l'élément de serrage (18) et serre un couvercle de cylindre (17) contre la plaque de vanne (16).
